# EUROPEAN PATENT APPLICATION

(11) **EP 4 201 916 A1**
(43) Date of publication of application: **28.06.2023**
(21) Application number: 22203188.2
(22) Date of filing: 24.10.2022
(51) Int. Cl.: C05F 11/08, A01N 63/22, A01N 63/20

(54) **SOIL IMPROVING AGENT AND PRODUCTION METHOD THEREFOR**

(30) Priority: 23.12.2021 JP 2021209276; 09.05.2022 JP 2022076920
(71) Applicant: Space Capital Co., Ltd., Hyogo 673-0443 (JP)
(72) Inventor: IZUMI, Takanori, Hyogo, 673-0443 (JP)
(74) Representative: Müller-Boré & Partner Patentanwälte PartG mbB

(57) **Abstract**

Provided is a soil improving agent formed in a pellet shape and having an excellent pathogenic microbe/virus eliminating ability. This soil improving agent formed in a pellet shape includes lactic acid bacteria being live cells and a pellet-forming carrier, and the pellet-forming carrier has a volume mean diameter of 50 to 130 µm.

## Description

### TECHNICAL FIELD

The present invention relates to a soil improving agent and a production method therefor.

### BACKGROUND ART

Lactic acid bacteria are known to have a soil improving function of changing soil into an environment in which plants easily grow. Therefore, liquid-state soil improving agents that contain lactic acid bacteria are widely used. For example, as a liquid-state soil improving agent/deodorant that contains lactic acid bacteria, KENKOU-DAICHI (manufactured by SPACE CAPITAL CO., LTD) is already commercially available. In addition, lactic acid bacteria are known to have a function (hereinafter, referred to as "pathogenic microbe/virus eliminating ability") of eliminating pathogenic microbes and viruses due to a strong acid of PH4 or lower generated by the lactic acid bacteria. Accordingly, root rot, diseases, and the like of crops are improved and prevented.

Soil improving agents that contain lactic acid bacteria and formed in a pellet-shape are publicly known. For example, PATENT LITERATURE 1 discloses a fertilizer having reduced offensive odor, obtained by: adding a phosphoric acid and a potassium substance to soybean curd refuse treated with lactic acid bacteria; processing the resultant matter into pellets; and drying the pellets. PATENT LITERATURE 2 discloses a soil improving agent obtained by: blending a bamboo powder, a pest repellent composition, and an organic fertilizer together; adding effective microorganisms such as lactic acid bacteria; and fermenting the resultant mixture. PATENT LITERATURE 3 discloses a compost production method in which bacteria-carrying sewage sludge residue pellets obtained by causing sewage sludge residue pellets to carry Bacillus bacteria therein and to carry lactic acid bacteria on a surface layer part thereof, are fermented.

PATENT LITERATURE 4 discloses a preparation method for an organic fertilizer for potted flowers as below. That is, the preparation method includes (1) mixing sheep dung, cow dung, and a yeast to acquire a primary fermented material, (2) degrading by a complex enzyme a plant particle carrier passed through a 40 to 60 mesh, to acquire an enzymolysis plant particle carrier, (3) mixing the primary fermented material, the enzymolysis plant particle carrier, and lactic acid bacteria together to acquire a secondary fermented material, (4) microwave freeze-drying the secondary fermented material at a heating temperature of 97 to 98°C at a microwave frequency of 1.5 to 1.7 MHz, to acquire a semidried matter, (5) pulverizing the semidried matter so as to have a particle diameter of 30 to 40 µm, and then pelletizing the resultant matter into pellets having a particle diameter of 1.6 to 1.8 mm, to acquire an organic fertilizer for potted flowers, and (6) performing packaging.

### CITATION LIST

### [PATENT LITERATURE]

PATENT LITERATURE 1: Japanese Laid-Open Patent Publication No. H10-327764
PATENT LITERATURE 2: Japanese Laid-Open Patent Publication No. 2009-24141
PATENT LITERATURE 3: Japanese Patent No. 6528188
PATENT LITERATURE 4: Chinese Laid-Open Patent Publication No 105948846

### SUMMARY OF THE INVENTION

### [TECHNICAL PROBLEM]

Conventional liquid-state soil improving agents that contain lactic acid bacteria need to be, for example, diluted, or sprayed by a sprayer at the time of use. Therefore, such liquid-state soil improving agents are low in usability when people enjoy kitchen gardens in a carefree manner while using planters, for example.

The soil improving agents described in PATENT LITERATURES 1 to 3 are formed in pellet shapes. Therefore, when a kitchen garden is made by using a planter, it is only necessary to load a pellet-shaped soil improving agent that contains lactic acid bacteria into a planter, and thus, usability is improved. However, the present inventors found that conventional soil improving agents formed in pellet shapes are low in the pathogenic microbe/virus eliminating ability. Therefore, conventional soil improving agents formed in pellet shapes may fail to sufficiently improve or prevent root rot or diseases.

In the preparation method for the organic fertilizer for potted flowers described in PATENT LITERATURE 4, in step (4) of PATENT LITERATURE 4 described above, microwave freeze-drying treatment is performed on the secondary fermented material that contains lactic acid bacteria. The microwave freeze-drying treatment in step (4) above has a bactericidal action. That is, in the case of the preparation method for the organic fertilizer for potted flowers described in PATENT LITERATURE 4, the lactic acid bacteria in the secondary fermented material are sterilized in step (4) above. Therefore, the organic fertilizer for potted flowers acquired through pelletization in step (5) of PATENT LITERATURE 4 described above is considered not to be able to exhibit a soil improving function due to the lactic acid bacteria or the pathogenic microbe/virus eliminating ability.

An object of the present invention is to provide a soil improving agent that has an excellent pathogenic microbe/virus eliminating ability and that is formed in a pellet shape. Another object of the present invention is to provide a production method for the soil improving agent above.

### [SOLUTION TO PROBLEM]

The present invention is a soil improving agent formed in a pellet shape, the soil improving agent including lactic acid bacteria and a pellet-forming carrier, wherein the pellet-forming carrier has a volume mean diameter of 50 to 130 µm.

The present invention is a production method for a soil improving agent formed in a pellet shape, the production method including: a grinding step of grinding a pellet-forming carrier so as to have a volume mean diameter of 50 to 130 µm; an attachment step of attaching lactic acid bacteria to the pellet-forming carrier prepared in the grinding step; and a pelletization step of pelletizing the pellet-forming carrier prepared in the attachment step and having attached thereto the lactic acid bacteria, into a pellet shape.

### [ADVANTAGEOUS EFFECTS OF THE INVENTION]

The soil improving agent formed in a pellet shape of the present invention has an excellent pathogenic microbe/virus eliminating ability.

### DETAILED DESCRIPTION

Hereinafter, the present disclosure will be described in detail with reference to a preferred embodiment.
(1) An embodiment of the present disclosure is a soil improving agent formed in a pellet shape, the soil improving agent including:
   lactic acid bacteria being live cells; and
   a pellet-forming carrier, wherein
   the pellet-forming carrier has a volume mean diameter of 50 to 130 µm.
   The soil improving agent formed in a pellet shape of (1) above has an excellent pathogenic microbe/virus eliminating ability.
(2) In the soil improving agent formed in a pellet shape according to (1) above, the lactic acid bacteria are ones belonging to genus Lactobacillus.
(3) In the soil improving agent formed in a pellet shape according to (1) or (2) above, the lactic acid bacteria are lactic acid bacteria derived from a plant.
(4) In the soil improving agent formed in a pellet shape according to (3) above, the plant is wormwood.
(5) In the soil improving agent formed in a pellet shape according to any one of (1) to (4) above, the pellet-forming carrier includes at least one selected from coconut fibers and sawdust.
   The soil improving agent formed in a pellet shape according to (2) to (5) above has a further excellent pathogenic microbe/virus eliminating ability.
(6) The soil improving agent formed in a pellet shape according to any one of (1) to (5) above further includes at least one selected from photosynthetic bacteria, yeasts, actinomycetes, and acetic acid bacteria.
   The soil improving agent formed in a pellet shape according to (6) above has an excellent deodorant ability in addition to the excellent pathogenic microbe/virus eliminating ability.
(7) The soil improving agent formed in a pellet shape according to any one of (1) to (6) above has a number of live cells of 10⁶ to 10⁷ CFU/g.
   The soil improving agent formed in a pellet shape according to (6) above has a further excellent pathogenic microbe/virus eliminating ability.
(8) A production method for a soil improving agent formed in a pellet shape includes:
   a grinding step of grinding a pellet-forming carrier such that the pellet-forming carrier has a volume mean diameter of 50 to 130 µm;
   an attachment step of attaching lactic acid bacteria being live cells to the pellet-forming carrier prepared in the grinding step; and
   a pelletization step of pelletizing the pellet-forming carrier prepared in the attachment step and having attached thereto the lactic acid bacteria, into a pellet shape.

The production method of (8) above can produce a soil improving agent, formed in a pellet shape, that has an excellent pathogenic microbe/virus eliminating ability.

The soil improving agent (hereinafter, referred to as "pellet soil improving agent") formed in a pellet shape according to an embodiment of the present disclosure includes lactic acid bacteria and a pellet-forming carrier, arid the pellet-forming carrier has a volume mean diameter of 50 to 130 µm. The maximum diameter of the pellet soil improving agent itself is preferably 3 to 10 mm, particularly preferably 4 to 8 mm. Accordingly, handling of the soil improving agent is facilitated.

The lactic acid bacteria of the present embodiment are ones belonging to at least one selected from the genus Lactobacillus, the genus Bifidobacterium, the genus Enterococcus, the genus Lactococcus, the genus Pediococcus, and the genus Leuconostoc. As the lactic acid bacteria of the present embodiment, ones belonging to the genus Lactobacillus are preferable. When lactic acid bacteria belonging to the genus Lactobacillus are used, the pathogenic microbe/virus eliminating ability of the pellet soil improving agent above is further improved.

The lactic acid bacteria described above is preferably lactic acid bacteria derived from a plant. The lactic acid bacteria derived from a plant are stronger, in a severe environment such as intestines of an animal, than lactic acid bacteria living in a certain environment. In particular, the lactic acid bacteria derived from a plant are excellent in acid tolerance and salt tolerance. Further, since the lactic acid bacteria derived from a plant are ones that are present in the natural world, the lactic acid bacteria are less likely to cause unbalance in the natural world or inhibit activities of other fermentative bacteria. Examples of the plant include wormwood, bamboo, and Pueraria lobate. As the lactic acid bacteria of the present embodiment, lactic acid bacteria derived from wormwood are preferable. By use of the lactic acid bacteria derived from wormwood, the pathogenic microbe/virus eliminating ability of the pellet soil improving agent above is further improved.

The pellet soil improving agent of the present embodiment can further include at least one selected from photosynthetic bacteria, yeasts, actinomycetes, and acetic acid bacteria (hereinafter, these may be referred to as "photosynthetic bacteria, etc."), in addition to the lactic acid bacteria described above. When the pellet soil improving agent further includes the photosynthetic bacteria, etc., the deodorant ability of the pellet soil improving agent is improved. The photosynthetic bacteria, etc. are preferably derived from a plant, similar to the lactic acid bacteria described above. In particular, photosynthetic bacteria, etc. that are derived from the same plant as that of the lactic acid bacteria included in the pellet soil improving agent are preferable. Accordingly, in the pellet soil improving agent above, the lactic acid bacteria and the photosynthetic bacteria, etc. can appropriately live in symbiosis.

Symbiosis between the lactic acid bacteria and the photosynthetic bacteria in the pellet soil improving agent above can effectively enhance the deodorant ability. Examples of specific photosynthetic bacteria to be caused to live in symbiosis with the lactic acid bacteria include Rhodopseudomonas capsulata, Rhodopseudomonas sphaeroides, Rhodospirillum rubrum, and Chromatium vinosum. In the pellet soil improving agent of the present embodiment, as the photosynthetic bacteria to be caused to live in symbiosis with the lactic acid bacteria, Rhodopseudomonas capsulata is preferable. Accordingly, in the pellet soil improving agent above, the deodorant ability is further improved in addition to the pathogenic microbe/virus eliminating ability.

It is preferable that the lactic acid bacteria and the photosynthetic bacteria, etc. described above are present in a state of live cells. The above live cells in the pellet soil improving agent settle in the soil, whereby the soil improving effect, the deodorant effect, and the pathogenic microbe/virus eliminating effect are maintained. The number of live cells in the pellet soil improving agent is preferably 10⁶ to 10⁷ CFU/g. In particular, preferably, the number of live lactic acid bacteria in the pellet soil improving agent is 2×10⁶ to 9×10⁶. When the number of live cells in the pellet soil improving agent is not less than 10⁶, live cells in the pellet soil improving agent can sufficiently settle in the soil. When the number of live cells in the pellet soil improving agent is not greater than 10⁷, the pellet-forming carrier described later can more appropriately hold the lactic acid bacteria as live cells. From the viewpoint of settlement to the soil and proliferation of the lactic acid bacteria and the photosynthetic bacteria, etc. in the pellet soil improving agent, it is preferable that the pellet soil improving agent is periodically loaded to the soil.

The pellet-forming carrier of the present embodiment may be any material that can be used for the pellet soil improving agent, and is not limited in particular. Specific examples of the pellet-forming carrier include sawdust, coconut fibers, zeolite, wood chips, chaffs, peat moss, resin beads, palm fibers, plastic fibers, charcoal, and crab shells. The pellet-forming carrier included in the pellet soil improving agent of the present embodiment may be of a single type or two or more types.

The pellet-forming carrier described above preferably includes at least one selected from sawdust and coconut fibers. Each of sawdust and coconut fibers can appropriately hold the above-described lactic acid bacteria as live cells. When the pellet soil improving agent includes sawdust as the pellet-forming carrier, the pellet soil improving agent has a light-brown-like color. Therefore, when a kitchen garden using a planter is enjoyed, the kitchen garden provides a light impression. When the pellet soil improving agent includes coconut fibers as the pellet-forming carrier, the water-holding capacity of the pellet soil improving agent is increased. Accordingly, growth of plants is promoted.

The pellet-forming carrier included in the pellet soil improving agent of the present embodiment has a volume mean diameter of 50 to 130 µm, preferably 60 to 120 µm. The volume mean diameter of the pellet-forming carrier can be measured by a commercially available microtrac particle size distribution analyzer. More specifically, the volume mean diameter of the pellet-forming carrier is measured by using a microtrac particle size distribution analyzer, MICROTRAC MT-3000 VSR (manufactured by Nikkiso Co., Ltd.), on the basis of an instruction manual thereof. When the volume mean diameter of the pellet-forming carrier included in the pellet soil improving agent is set to 50 to 130 µm, in particular 60 to 120 µm, the pathogenic microbe/virus eliminating ability of the pellet soil improving agent is further improved.

A production method of the pellet soil improving agent according to another embodiment of the present disclosure includes: a grinding step of grinding the pellet-forming carrier so as to have a volume mean diameter of 50 to 130 µm; an attachment step of attaching the lactic acid bacteria to the pellet-forming carrier prepared in the grinding step; and a pelletization step of pelletizing the pellet-forming carrier prepared in the attachment step and having attached thereto the lactic acid bacteria, into a pellet shape. It should be noted that the terms that are used in common in the description of the production method for the pellet soil improving agent according to another embodiment and in the description of the pellet soil improving agent according to the embodiment described above have the same meanings as those described in the pellet soil improving agent according to the embodiment described above.

In the grinding step of the present embodiment, any technique, which is not limited in particular, that can crush the pellet-forming carrier so as to have a volume mean diameter of 50 to 130 µm can be used. For example, grinding of the pellet-forming carrier can be performed by a grinder such as a cutter mill, a high-speed rotation mill, a ball mill, a rod mill, a hammer mill, or a roller mill. Among commercially available pellet production apparatuses, there is a pellet production apparatus that can independently use a function of crushing materials associated with pelletization. When the pellet-forming carrier is crushed a plurality of times by using this crushing function, it is also possible to crush the pellet-forming carrier so as to have a volume mean diameter of 50 to 130 µm. In the grinding step, the pellet-forming carrier is preferably ground such that the pellet-forming carrier after the grinding step has a volume mean diameter of 60 to 120 µm.

In the attachment step of the present embodiment, any attaching technique, which is not limited in particular, that can attach the above-described lactic acid bacteria to the crushed pellet-forming carrier can be used. For example, a technique (technique A) in which a liquid containing the lactic acid bacteria is sprayed to the crushed pellet-forming carrier by using a sprayer, a technique (technique B) in which a liquid containing the lactic acid bacteria is caused to infiltrate the crushed pellet-forming carrier, a technique (technique C) in which the lactic acid bacteria that are alive and in a powder form and the pellet-forming carrier are mixed together, or the like can be used. In the technique A, the use amount of the liquid containing the lactic acid bacteria can be reduced, compared with the technique B. In the technique A, the mixing work, which is necessary for the technique C, can be omitted. Therefore, as the attachment step, from the viewpoint of efficiency, the technique A in which a liquid containing the lactic acid bacteria is sprayed is preferable.

In the attachment step described above, in addition to the lactic acid bacteria, the above-described photosynthetic bacteria, etc. may also be attached to the crushed pellet-forming carrier. As an attaching technique, the same technique as that for the lactic acid bacteria can be used. For example, in a case where the technique A described above is used, a liquid containing the lactic acid bacteria and the photosynthetic bacteria, etc. is sprayed to the crushed pellet-forming carrier, whereby the lactic acid bacteria and the photosynthetic bacteria, etc. can be attached to the crushed pellet-forming carrier. Alternatively, a liquid containing the lactic acid bacteria and a liquid containing the photosynthetic bacteria, etc. may be separately attached to the pellet-forming carrier. From the viewpoint of simplification of the attachment step, the above-described attachment step using a liquid containing the lactic acid bacteria and the photosynthetic bacteria, etc. is preferable. In this case, from the viewpoint of symbiosis between the lactic acid bacteria and the photosynthetic bacteria, etc., the lactic acid bacteria and the photosynthetic bacteria, etc. contained in the above liquid are preferably those derived from the same plant. As a more specific example of the attachment step, a liquid containing lactic acid bacteria and photosynthetic bacteria, etc. that have been extracted from wormwood may be sprayed to the crushed pellet-forming carrier by using a sprayer.

In the pelletization step of the present embodiment, any known pelletization technique, which is not limited in particular, may be used to pelletize the pellet-forming carrier prepared in the attachment step described above and having attached thereto the lactic acid bacteria. For example, the above pellet-forming carrier having attached thereto the lactic acid bacteria can be pelletized by using a commercially available pellet production apparatus.

### [Examples]

In the following, the present disclosure will be further specifically described with reference to Examples. However, the present disclosure is not limited to Examples.

### [Pellet-forming carrier]

As the pellet-forming carrier, sawdust (manufactured by Shisou-no Mori-no-ki) and coconut fibers (CoCo natural: manufactured by SPACE CAPITAL CO., LTD) were used.

### [Lactic acid bacteria-containing liquid]

As a lactic acid bacteria-containing liquid, KENKOU-DAICHI (manufactured by SPACE CAPITAL CO., LTD) was used. The lactic acid bacteria contained in KENKOU-DAICHI are lactic acid bacteria derived from wormwood.

### [Coarsely ground wood]

The above-mentioned sawdust purchased from Shisou-no Mori-no-ki was used as coarsely ground wood.

### [Coarsely ground coconut]

The above- mentioned CoCo natural manufactured by SPACE CAPITAL CO., LTD was used as coarsely ground coconut.

### [Powder wood]

Powder wood was prepared by grinding sawdust under a condition of a screen (mesh) of 1.8 mm, by using a grinder, that is, a cutter mill (manufactured by SANRIKI SEISAKUSHO.Co., Ltd.).

### [Powder coconut]

Powder coconut was prepared by the same method as that in the preparation of the powder wood described above, except that coconut fibers were used instead of the sawdust.

### [Measurement of particle size distribution]

The coarsely ground wood, the coarsely ground coconut, the powder wood, and the powder coconut were each caused to pass through a 1.4 mm sieve mesh, and with respect to each sample having passed through the sieve, the particle size distribution was measured. With respect to the coarsely ground wood, the coarsely ground coconut, the powder wood, and the powder coconut, the mass% of particles of 1.4 mm or larger that did not pass through the 1.4 mm sieve mesh is shown in Table 1.

**[Table 1]**

| | Particle of 1.4 mm or larger (unit: mass%) |
|---|---|
| Coarsely ground wood | 27.5 |
| Coarsely ground coconut | 8.6 |
| Powder wood | 0 |
| Powder coconut | 0 |

Table 2 shows the measurement results of the particle size distribution. The measurement apparatus and the measurement condition are shown below.
Measurement apparatus: microtrac particle size distribution analyzer MICROTRAC MT-3000 VSR (manufactured by Nikkiso Co., Ltd.)
Measurement principle: laser diffraction/scattering
Measurement range: 0.021 to 1408 µm
Measurement time: 30 seconds
Solvent used: methanol

**[Table 2]**

| | Coarsely ground wood | Coarsely ground coconut | Powder wood | Powder coconut |
|---|---|---|---|---|
| Volume mean diameter: mv (µm) | 294.8 | 227.9 | 107.6 | 68.73 |
| Number mean diameter: mn (µm) | 19.18 | 24.56 | 21.88 | 19.41 |
| Surface mean diameter: ma (µm) | 103.1 | 108 | 62.16 | 44.79 |
| Calculated specific surface area: cs (m²/cm³) | 0.058 | 0.056 | 0.097 | 0.134 |
| Standard deviation: sd | 247.9 | 167.7 | 67.6 | 36.52 |

### [Comparative example 1]

The coarsely ground wood was pelletized by using a pellet production apparatus, that is, pelletizer HG-ZLSP150B (manufactured by Haige Industry Co., Ltd.), on the basis of an instruction manual. The prepared pelletized formed matter was used as Comparative example 1.

### [Comparative example 2]

Pelletized formed matter was prepared by the same method as in Comparative example 1 except that the coarsely ground coconut was used instead of the coarsely ground wood. The prepared pelletized formed matter was used as Comparative example 2.

### [Comparative example 3]

Pelletized formed matter was prepared by the same method as in Comparative example 1 except that the powder wood was used instead of the coarsely ground wood. The prepared pelletized formed matter was used as Comparative example 3.

### [Comparative example 4]

Pelletized formed matter was prepared by the same method as in Comparative example 1 except that the powder coconut was used instead of the coarsely ground wood. The prepared pelletized formed matter was used as Comparative example 4.

### [Comparative example 5]

10 ml of the lactic acid bacteria-containing liquid was sprayed to 100 g of the coarsely ground wood by using a sprayer. The coarsely ground wood having the lactic acid bacteria-containing liquid sprayed thereto was pelletized by using a pellet production apparatus, that is, pelletizer HG-ZLSP150B (manufactured by Haige Industry Co., Ltd.), on the basis of an instruction manual, whereby pelletized formed matter was prepared. The prepared pelletized formed matter was used as Comparative example 5.

### [Comparative example 6]

Pelletized formed matter was prepared by the same method as in Comparative example 5 except that the coarsely ground coconut was used instead of the coarsely ground wood. The prepared pelletized formed matter was used as Comparative example 6.

### [Example 1]

Pelletized formed matter was prepared by the same method as in Comparative example 5 except that the powder wood was used instead of the coarsely ground wood. The prepared pelletized formed matter was used as Example 1.

### [Example 2]

Pelletized formed matter was prepared by the same method as in Comparative example 5 except that the powder coconut was used instead of the coarsely ground wood. The prepared pelletized formed matter was used as Example 2.

### [Bacteria elimination effect test]

E. coli (NBRC3301) was cultured in a nutrient agar medium at 37°C±2°C for 18 to 24 hours. Then, the number of bacteria was adjusted to be 10⁴ CFU/ml, and the resultant matter was used as a test bacteria liquid. 10 g of the pelletized formed matter of Comparative example 1 was suspended in 90 ml of saline, and the resultant matter was used as a test specimen of Comparative example 1. Similar to the test specimen of Comparative example 1, a test specimen of each of Comparative examples 2 to 6, Example 1, and Example 2 was prepared.

0.1 ml of the test bacteria liquid was added to the test specimen of each of Comparative examples 1 to 6, Example 1, and Example 2. The number of E. coli of each test specimen at the time point (after 0 hours) of the addition of 0.1 ml of the test bacteria liquid thereto, and the number of E. coli of each test specimen after the test specimen was left to stand for 24 hours at room temperature were measured by using a desoxycholate agar medium. Table 3 shows the measurement results of the number of E. coli.

**[Table 3]**

| | After 0 hours (CFU/ml) | After 24 hours (CFU/ml) |
|---|---|---|
| Comparative example 1 | 91 | >300 |
| Comparative example 2 | 82 | >300 |
| Comparative example 3 | 105 | >300 |
| Comparative example 4 | 105 | >300 |
| Comparative example 5 | 151 | <60 |
| Comparative example 6 | 97 | <70 |
| Example 1 | 95 | <30 |
| Example 2 | 99 | <40 |

Table 3 reveals that, in Comparative examples 1 to 4 in which the lactic acid bacteria-containing liquid was not sprayed, the number of E. coli 24 after hours increased relative to that after 0 hours. Meanwhile, in Comparative example 5, Comparative example 6, Example 1, and Example 2 in which the lactic acid bacteria-containing liquid was sprayed, it is seen that the number of E. coli after 24 hours decreased relative to that after 0 hours. This suggests that the pellet soil improving agent containing the lactic acid bacteria has the pathogenic microbe/virus eliminating ability.

The ratio of the number of E. coli after 24 hours relative to that after 0 hours was about 40% in Comparative example 5, was 32% in Example 1, was about 72% in Comparative example 6, and was 40% in Example 2. Each of Comparative example 5 and Example 1 is a pellet soil improving agent in which sawdust was used as the pellet-forming carrier. Each of Comparative example 6 and Example 2 is a pellet soil improving agent in which coconut fibers were used as the pellet-forming carrier. Here, as shown in Table 2 described above, the sizes of particles of the pellet-forming carrier are larger in Comparative example 5 and Comparative example 6 than in Example 1 and Example 2. More specifically, the volume mean diameters of particles of Comparative example 5 and Comparative example 6 are 294.8 µm and 227.9 µm, respectively. Meanwhile, the volume mean diameters of particles of Example 1 and Example 2 are 107.6 µm and 68.73 µm, respectively. That is, Table 3 reveals that, in the cases of the pellet soil improving agents, containing the lactic acid bacteria, in which the same pellet-forming carrier is used, the pellet soil improving agent that contains a pellet-forming carrier having a larger size of particles has a smaller number of E. coli after 24 hours. From this, it is experimentally suggested that setting the volume mean diameter of the pellet-forming carrier contained in the pellet soil improving agent to 50 to 130 µm improves the pathogenic microbe/virus eliminating ability.

### [Live cell number measurement test]

10 g of the pelletized formed matter of Example 1 was suspended in 90 ml of saline and the resultant matter was used as a live cell number measurement sample 1. Using saline, the prepared live cell number measurement sample 1 was 10-fold diluted, 100-fold diluted, and 1000-fold diluted, whereby three diluted samples were prepared. The live cell number measurement sample 1 was cultured by using an MRS agar medium conforming to International Standard NF ISO 15214, at 30°C for 72 hours, and the colonies after the culture were measured. Similar to the live cell number measurement sample 1, the above three diluted samples were each cultured by using an MRS agar medium, and the colonies after the culture were measured. On the basis of the measurement result of the colonies, the number of live cells in the pelletized formed matter of Example 1 was calculated.

10 g of the pelletized formed matter of Example 2 was suspended in 90 ml of saline, and the resultant matter was used as a live cell number measurement sample 2. The number of live cells in the pelletized formed matter of Example 2 was calculated by the same technique as that in the live cell number measurement test using the live cell number measurement sample 1 described above, except that the live cell number measurement sample 2 was used. Table 4 shows calculation results of the number of live cells regarding the live cell number measurement sample 1 and the live cell number measurement sample 2.

**[Table 4]**

| | Number of live cells (CFU/g) |
|---|---|
| Live cell number measurement sample 1 | 8300000 |
| Live cell number measurement sample 2 | 2200000 |

Table 4 shows that the pelletized formed matters of Example 1 and Example 2 which have excellent pathogenic microbe/virus eliminating abilities have a number of live cells of 10⁶ to 10⁷ CFU/g. The MRS agar medium is a selective medium that urges growth of the lactic acid bacteria. Therefore, from Table 4, it is experimentally suggested that the pelletized formed matters of Example 1 and Example 2 hold the lactic acid bacteria of 2×10⁶ to 9×10⁶ CFU/g as live cells.

## Claims

1. A soil improving agent formed in a pellet shape, the soil improving agent comprising:
lactic acid bacteria being live cells; and
a pellet-forming carrier, wherein
the pellet-forming carrier has a volume mean diameter of 50 to 130 µm.

2. The soil improving agent formed in a pellet shape according to claim 1, wherein the lactic acid bacteria are ones belonging to genus Lactobacillus.

3. The soil improving agent formed in a pellet shape according to claim 1 or 2, wherein
the lactic acid bacteria are lactic acid bacteria derived from a plant.

4. The soil improving agent formed in a pellet shape according to claim 3, wherein the plant is wormwood.

5. The soil improving agent formed in a pellet shape according to claim 1, wherein
the pellet-forming carrier includes at least one selected from coconut fibers and sawdust.

6. The soil improving agent formed in a pellet shape according to claim 1, further including
at least one selected from photosynthetic bacteria, yeasts, actinomycetes, and acetic acid bacteria.

7. The soil improving agent formed in a pellet shape according to claim 1, 2, 5, or 6, having a number of live cells of 10⁶ to 10⁷ CFU/g.

8. A production method for a soil improving agent formed in a pellet shape, the production method comprising:
a grinding step of grinding a pellet-forming carrier such that the pellet-forming carrier has a volume mean diameter of 50 to 130 µm;
an attachment step of attaching lactic acid bacteria being live cells to the pellet-forming carrier prepared in the grinding step; and
a pelletization step of pelletizing the pellet-forming carrier prepared in the attachment step and having attached thereto the lactic acid bacteria, into a pellet shape.
